# EUROPEAN PATENT SPECIFICATION

(11) **EP 0 414 701 B1**
(45) Date of publication and mention of the grant of the patent: **27.09.1995**
(21) Application number: 89904141.2
(22) Date of filing: 11.03.1989
(51) Int. Cl.: A61L 15/07, C08G 18/48, C08G 18/76

(54) **ORTHOPAEDIC MATERIAL OF ISOCYANATE RESIN**
ORTHOPÄDISCHES MATERIAL AUS ISOCYANATSTOFF
MATERIAU ORTHOPEDIQUE EN RESINE D'ISOCYANATE

(30) Priority: 11.03.1988 GB 8805827; 01.07.1988 GB 8815734
(43) Date of publication of application: 06.03.1991
(73) Proprietor: SMITH & NEPHEW P.L.C., London WC2R 3BP (GB)
(72) Inventor: HICKS, John, Kenneth, Hull, Humberside (GB)
(74) Representative: Gilholm, Stephen Philip
(86) International application number: GB8900249
(87) International publication number: WO8908463

(56) References cited:
- EP-A- 0 086 621
- US-A- 0 172 379
- US-A- 4 285 073
- US-A- 4 377 645
- US-A- 4 427 003
- US-A- 4 547 561

## Description

The present invention relates to a hardenable material which comprises a substrate which carries an isocyanate functional resin which hardens on contact with water. More specifically, this invention relates to an orthopaedic splint bandage which comprises a substrate which carries low tack isocyanate function resin which hardens on contact with water to form a strong splint.
In US Patent No. 4377645 there is disclosed a polyurethane sponge having the properties of a cellulose foam sponge, in particular the water-holding and wiping ability. Such sponges are formed by the reaction of a poly (oxy C₂₋₄ alkylene) diol, a methylene-bis (phenyl isocyanate) containing isocyanate product and a monomeric cross-linking agent having 3 or 4 hydroxyl equivalents per mole. Such sponges are flexible materials since they are stated to be able to be squeezed by hand to release absorbed water.

EP-A-0086621 discloses a polyurethane cast bandage comprising a fibrous substrate coated with polyurethane prepolymer formed from an aromatic isocyanate and a polyol. Stated polyols include polyether polyols and polyester polyols.

UK Patent No. 2092606B discloses splinting bandages which comprise a substrate which carries an isocyanate functional resin which hardens on contact with water. The patent points out that hydrophilic components such as polyethylenoide condensates, for example polyethylene glycols or polyoxyethylene sorbitan esters, can be employed to make resins. Use of resins incorporating such components leads to a product which has low tack after immersion in water. A difficulty encountered with these products in that they can have a low strength in the period immediately following immersion in water and application although after some time they do form an acceptable splint or cast. A method has now been found by which resins can be made which are intrinsically low tack and which also forms strong splints or casts shortly after immersion in water and application. Thus instead of having to wait until some hours for a major cast to become load-bearing the patient has the possibility of walking on a cast in 30 minutes or even less. It has generally been possible to achieve short setting times by increasing the amount of catalyst present in the resins but this then tends to shorten the working time of the splinting material (that is the time after wetting that the material is sufficiently flexible to be moulded about the part of the body to be immobilised and can lead to a product in which the exotherm is too high).

The present invention provides an orthopaedic splinting material which comprises a substrate which carries an isocyanate functional resin containing residues of (a) polyethylene glycol, (b) a triol or tetrol of molecular weight less than 200 and (c) an aromatic polyisocyanate in which resin the weight ratio of (a+b) to c is less than 1:1.

The splinting material is preferably a bandage but other forms of sheet materials, for example those to form slabs, are also envisaged.

Suitable triols or tetrols include glycerol, trimethylol propane, triethanolamine, pentaerythritol and the like. Particularly suitable triols or tetrol components are glycerol and trimethylol propane or mixtures thereof. A preferred triol component is glycerol. Another preferred triol component is trimethylol propane.

The molecular weight of the polyethylene glycol may be any which is convenient or may be a mixture of molecular weights (in which case it is the average molecular weight which is referred to hereinafter unless otherwise stated). Generally the molecular weight should be from 600 to 3500, for example about 1000. An average molecular weight of 700 - 1100, especially 800 - 900 is particularly desirable as excellent resins result, molecular weights in this range can be obtained by mixing high (eg 3000) and low (eg 600) molecular weight polyethylene glycols. Generally the molar ratio of polyethylene glycol to triol or tetrol will be from 2:1 to 1:2, more aptly 4:3 to 3:4 and very suitably about 1:1. Most aptly the polyethylene glycol comprises 20-30% by weight of the resin, preferably 22-28% by weight of the resin, for example 24-26% wt/wt of the resin.

The isocyanate will be a polyisocyanate and most suitably will be a diisocyanate optionally in the presence of its higher homologues or condensation products. The resin of this invention will most aptly contain 55-75% by weight of residues of aromatic isocyanate and preferably 60-72% by weight of residues of aromatic isocyanate, for example 70%. The favoured aromatic isocyanate for use in this invention is MDI optionally in admixture with its higher homologues. A suitable commercial grade of this is available as Isonate 143L or M143. Such MDI derived mixtures are preferred.

In the resin of this invention all or part of the aromatic isocyanate may have been incorporated into the form of a prepolymer in which it caps the polyethylene glycol or triol or tetrol.

The resins of this invention will usually have been formed by including water in the reaction mixture. This is normally found as dampness in the polyethethlene glycol and/or triol or tetrol, for example as 0.1 to 1.5% wt/wt of total polyols, more usually 0.2 to 1% wt/wt of total polyols.

Generally the resin will consist essentially of the residues of (a), (b) and (c) referred to hereinbefore (although minor amounts of other diols or triols may be used as long as they are used in small quantities which are not sufficient to adversely change the properties of the resin) together with a tertiary amine catalyst and other conventional additives such as stabilizers, antifoams and the like.

The tertiary amine catalyst is aptly a bis-tertiary amine such as a bisethylether derivative such as a compound of the formula: 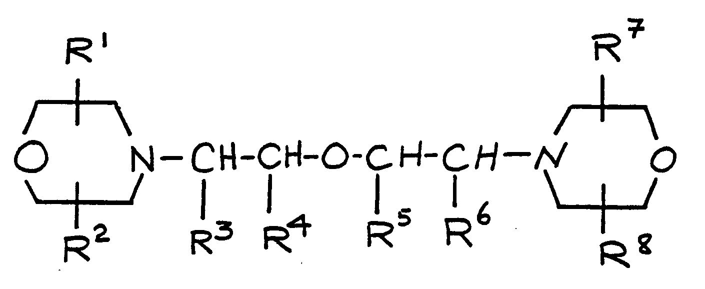
wherein R¹ to R⁸ are hydrogen atoms or methyl or ethyl groups. Aptly R¹ to R⁸ are hydrogen atoms or methyl groups. Favourably R¹ to R⁸ are hydrogen atoms. Favourably R¹, R², R³ and R⁸ are methyl groups and R³ to R⁶ are hydrogen atoms. Favourably R¹ to R⁴ and R⁶ to R⁸ are hydrogen atoms and R⁵ is a methyl group.

A preferred catalyst is di(2,6-dimethylmorpholino) diethylether.

The resin may comprise stabilizers such as sulphonic acid (for example methane or ethane sulphonic acid), succinic anhydride, benzoyl chloride or the like. The resin may also comprise other agents such as antifoams such as silicone oil, for example 0.5-5% and more usually 1-2% of silicone oil.

The amount of resin employed may be as desired but generally 40-50% of the total weight of resin plus substrate is resin.

The substrate may be any suitable substrate but knitted polymer or glass substrates are favoured of which glass substrates are preferred, for example a substrate such as in commercially available products such as Scotchcast or Dynacast. Other suitable substrates include woven and non-woven substrates, for example foams, apertured non-wovens and the like.

The resin may be prepared by placing the aromatic isocyanate in an reactor optionally together with any stabilizer such as benzoyl chloride, succinic anhydride or the like and antifoam, warming to about 55°C under dry nitrogen, adding thereto the polyethylene glycol and stirring for 1 hour at about 55°C, adding any additional stabilizers such as methane sulphonic or ethane sulphonic acids over five minutes and then adding the catalyst and stirring for a further 30 minutes. The resulting resin can be spread onto a substrate at the required weight.

The resins comprising components a, b and c as set forth hereinbefore with respect ot the splinting materials also form part of this invention.

The following Examples illustrate this invention.

### Example 1

The following formulation produces a low tack strong cast when spread on conventional knitted glass substrate at 44% mass weight =

| | |
|---|---|
| Isonate 143L | 2427.5g |
| PEG 1000 | 850.8g containing 0.1% water |
| Trimethylol Propane | 118.8g containing 1.45% water |
| Succinic Anhydride | 34.0g |
| Methane Sulphonic Acid | 1.0g |
| Antifoam | 34.0g |
| KL 26 | 34.0g |

Isonate 143L is aprtly polymerised MDI available from Dow (sometimes called M143). PEG 1000 is a polyethylene glycol of molecular weight 1000. The Antifoam is MSA silicone. KL 26 is bis(2,6-dimethylmorpholino) diethylether.

Charge the Isonate 143L and antifoam into a glass reactor and heated to 55°C. The ground succinic anhydride is added with agitation (agitation throughout hereafter). The PEG 1000 is added in 3 aliquots at 10 minute intervals and reacted for 30 minutes. The trimethylol propane is added in 3 aliquots at 10 minute intervals and reacted for 60 minutes. Methane sulphonic acid is added dropwise over 5 minutes and mixed for 10 minutes. The KL26 is added over 10 minutes and mixed for 10. the resin is then allowed to cool and discharged into a dried vessel which is then sealed.

This resin is spread at 25°C onto a suitable substrate under dry conditions (eg 2-5% RH). The resulting bandages were found to have coefficient of friction of 0.5 after immersion in water.

### Example 2

A resin was prepared using the following :

| | |
|---|---|
| Trimethylol Propane (+1.35% water) | 170.34g |
| Polyoxyethylene Glycol (+0.1% water) | 1224.54g |
| Isonate M143 | 3460.43g |
| Succinic Anhydride | 48.55g |
| Methane Sulphonic Acid | 1.46g |
| Antifoam | 48.55g |
| Di(dimethylmorpholino)diethylether | 46.13g |

The resin was coated onto a knitted glass substrate at 48% wt/wt. The PEG had a molecular weight of 1000. The antifoam was as in Example 1.

### Example 3

A resin was prepared analogous to that of Example 2 replacing the succinic anhydride with 24.27g of benzoyl chloride. The resin was coated onto a knitted glass substrate at 44% wt/wt.

### Example 4

A resin was prepared analogous to that of Example 2 replacing the trimethylol propane with an equivalent amount of glycerol. The resin was coated onto a knitted glass substrate at 45% wt/wt.

### Example 5

Resins were prepared and coated in Example 1 with the following formulation:

| | | |
|---|---|---|
| Isonate M143 | 10579.9g | 2977.7g |
| PEG 1000 | 3639.1g | - |
| Glycerol | 361.1g | 63.8g |
| Succinic Anhydride | 154.8g | 43.7g |
| Methane Sulphonic Acid | 4.4g | 1.3g |
| KL 26 | 123.9g | 7.2g |
| Silicone Oils | 745.8g | 43.7g |
| PEG 2000 | - | 1332.5g |

### Examples 6-10

Polyethylene glycols of 'nominal' molecular weights were prepared by blending the amounts of PEG 600 and PEG 3000 shown in the following Table 1.

**Table 1**

| PEG 600(gm) | PEG 3000 (g) | Nominal M.Wt | Example No |
|---|---|---|---|
| 8902 | 1098 | 700 | 6 |
| 7488 | 2512 | 800 | 7 |
| 6482 | 3518 | 900 | 8 |
| 5034 | 4966 | 1100 | 9 |
| 2525 | 7475 | 1600 | 10 |

The components shown in Table 2 were formed into resins by the following procedure:
The succinic anhydride, isocyanate, and antifoaming agent were charged to the reactor and heated over a water bath maintained at 50°C. When the temperature of the reactants had reached 45°C the PEG was added in two aliquots, the temperature rise being allowed to stabilize before addition of the second aliquot. The mixture was stirred for a further 20 minutes after which the glycerol was added and the mixture stirred for yet a further hour.

The methane sulphonic acid was added slowly and the mixture stirred for 10 minutes whereupon the catalyst was added. The whole reactant mass was stirred for 30 minutes, allowed to cool to ambient temperature and discharged from the reactor.

**Table 2**

| Example (gms) | 6 | 7 | 8 | 9 | 10 |
|---|---|---|---|---|---|
| Glycerol | 62.31 | 71.30 | 74.33 | 78.94 | 93.27 |
| PEG | 1164 | 1168 | 11761 | 1197 | 1202 |
| Isonate M143⁽¹⁾ | 3154 | 3141 | 3130 | 3105 | 3085 |
| Succinic Anhydride | 43.8 | 43.8 | 43.8 | 43.8 | 43.8 |
| Methane Sulphonic Acid | 1.31 | 1.31 | 1.31 | 1.31 | 1.31 |
| Anitfoam MSA | 43.8 | 43.8 | 43.8 | 43.8 | 43.8 |
| Catalyst⁽²⁾ | 30.66 | 30.66 | 30.66 | 30.66 | 30.66 |

| | | | | | |
|---|---|---|---|---|---|
| ⁽¹⁾diphenylmethane-4,4¹-diisocyanate containing polycarbodiimide adducts. | | | | | |
| ⁽²⁾bis (2,(-dimethyl morpholino-N-ethyl)ether. | | | | | |

Bandages were prepared by coating the resin onto a conventional fibre glass substrate and squeezing out the excess resin.

The resin coated substrates were then dipped into water and wound onto a mandrel, five layers thick to produce a number of casts. Each cast was removed from the mandrel and its strength determined, after the elapse of a predetermined period of time, by a flexural regidity test. The strength tests results are shown in Table 3 which also gives comparative results for a conventional splinting resin on the same substrate as that employed for the resins of the invention.

**Table 3**

| Example | strength at: | |
|---|---|---|
| | 15 mins | 30 mins |
| 6 | 2.59 | 2.87 |
| 7 | 2.62 | 3.13 |
| 8 | 2.46 | 2.90 |
| 9 | 1.91 | 1.63 |
| 10 | 1.91 | 2.17 |
| Comparative | 1.75 | 2.20 |

## Claims

1. An orthopaedic splinting material which comprises a substrate which carries an isocyanate functional resin containing residues of (a) polyethylene glycol, (b) a triol or tetrol of molecular weight less than 200 and (c) an aromatic polyisocyanate in which resin the weight ratio of (a)+(b) to (c) is less than 1:1.

2. An orthopaedic splinting material as claimed in claim 1 wherein the polyethylene glycol has a molecular weight of 600 to 3500.

3. An orthopaedic splinting material as claimed in claim 2 wherein the polyethylene glycol has a molecular weight of 700 to 1100 from a mixture of high and low molecular weight polyethylene glycols.

4. An orthopaedic splinting material as claimed in any of claims 1 to 3 wherein (b) is glycerol.

5. An orthopaedic splinting material as claimed in any of claims 1 to 3 wherein (b) is trimethylolpropane.

6. An orthopaedic splinting material as claimed in any of claims 1 to 5 which also comprises a tertiary amine catalyst.

7. An orthopaedic splinting material as claimed in any of claims 1 to 6 wherein the polyethylene glycol residues comprise 22-28% of the resin by weight.

8. An orthopaedic splinting material as claimed in any of claims 1 to 7 wherein the aromatic isocyanate residues comprise 60-72% of the resin by weight.

9. An orthopaedic splinting material as claimed in any of claims 1 to 8 wherein the substrate is a knitted glass substrate.

10. An orthopaedic splinting material as claimed in any one of claims 1 to 9 in the form of a bandage.

11. A resin which comprises components (a), (b), and (c) as set forth in any of claims 1 to 10, wherein the molar ratio of (a) : (b) is from 2:1 to 1:2.

## Patentansprüche

1. Orthopädisches Schienmaterial, welches ein Substrat umfaßt, das ein Harz mit Isocyanatgruppen trägt, das Reste (a) von Polyethylenglykol, (b) eines Triols oder Tetrols mit einem kleineren Molekulargewicht als 200 und (c) eines aromatischen Polyisocyanats enthält, wobei das Gewichtsverhältnis von (a) + (b) zu (c) in dem Harz kleiner als 1:1 ist.

2. Orthopädisches Schienmaterial wie in Anspruch 1 beansprucht, wobei das Polyethylenglykol ein Molekulargewicht von 600 bis 3500 besitzt.

3. Orthopädisches Schienmaterial wie in Anspruch 2 beansprucht, wobei das Polyethylenglykol ein Molekulargewicht von 700 bis 1100 ans einem Gemisch hoch- und niedermolekularer Polyethylenglykole besitz.

4. Orthopädisches Schienmaterial wie in einem der Ansprüche 1 bis 3 beansprucht, wobei (b) Glycerin ist.

5. Orthopädisches Schienmaterial wie in einem der Ansprüche 1 bis 3 beansprucht, wobei (b) Trimethylolpropan ist.

6. Orthopädisches Schienmaterial wie in einem der Ansprüche 1 bis 5 beansprucht, welches ferner ein tertiäres Amin als Katalysator enthält.

7. Orthopädisches Schienmaterial wie in einem der Ansprüche 1 bis 5 beansprucht, wobei die Polyethylenglykolreste 22-28 Gew.-% des Harzes umfassen.

8. Orthopädisches Schienmaterial wie in einem der Ansprüche 1 bis 7 beansprucht, wobei die aromatischen Isocyanatreste 60-72 Gew.-% des Harzes umfassen.

9. Orthopädisches Schienmaterial wie in einem der Ansprüche 1 bis 8 beansprucht, wobei das Substrat ein gewirktes Glassubstrat ist.

10. Orthopädisches Schienmaterial wie in einem der Ansprüche 1 bis 9 beansprucht in Form eines Verbandes.

11. Harz, welches die in einem der Ansprüche 1 bis 10 angegebenen Bestandteile (a), (b) und (c) enthält, wobei das Molverhältnis von (a):(b) 2:1 bis 1:2 beträgt.

## Revendications

1. Matériau pour contention orthopédique qui comprend un substrat qui porte une résine fonctionnelle d'isocyanate contenant des résidus de (a) un polyéthylène glycol, (b) un triol ou un tétrol de poids moléculaire inférieur à 200 et (c) un polyisocyanate aromatique, dans laquelle résine le rapport en poids de [(a) + (b)] à (c) est inférieur à 1:1.

2. Matériau pour contention orthopédique suivant la revendication 1, dans lequel le polyéthylène glycol a un poids moléculaire de 600 à 3500.

3. Matériau pour contention orthopédique suivant la revendication 2, dans lequel le polyéthylène glycol a un poids moléculaire de 700 à 1100 provenant d'un mélange de polyéthylène glycols de haut poids moléculaire et de bas poids moléculaire.

4. Matériau pour contention orthopédique suivant l'une quelconque des revendications 1 à 3, dans lequel (b) est le glycérol.

5. Matériau pour contention orthopédique suivant l'une quelconque des revendications 1 à 3, dans lequel (b) est le triméthylolpropane.

6. Matériau pour contention orthopédique suivant l'une quelconque des revendications 1 à 5, qui comprend aussi un catalyseur de type amine tertiaire.

7. Matériau pour contention orthopédique suivant l'une quelconque des revendications 1 à 6, dans lequel les résidus de polyéthylène glycol constituent 22 à 28% en poids de la résine.

8. Matériau pour contention orthopédique suivant l'une quelconque des revendications 1 à 7, dans lequel les résidus d'isocyanate aromatique constituent 60 à 72% en poids de la résine.

9. Matériau pour contention orthopédique suivant l'une quelconque des revendications 1 à 8, dans lequel le substrat est un substrat de verte tricoté.

10. Matériau pour contention orthopédique suivant l'une quelconque des revendications 1 à 9 sous la forme d'un bandage.

11. Résine qui comprend des composants (a), (b) et (c) tels que définis dans l'une quelconque des revendications 1 à 10, dans laquelle le rapport molaire de (a):(b) de 2:1 à 1:2.
